(19) Europäisches Patentamt
European Patent Office
Office européen des brevets

(11) Publication number: **0 079 991**
**B1**

(12) **EUROPEAN PATENT SPECIFICATION**

(45) Date of publication of patent specification: **26.02.86**

(51) Int. Cl.$^4$: **C 07 C 148/00**

(21) Application number: **81305520.9**

(22) Date of filing: **23.11.81**

(54) **Method for making thiobiscarbamates.**

(43) Date of publication of application:
**01.06.83 Bulletin 83/22**

(45) Publication of the grant of the patent:
**26.02.86 Bulletin 86/09**

(84) Designated Contracting States:
**BE CH DE FR GB IT LI LU NL**

(56) References cited:
**EP-A-0 025 354**

**The file contains technical information submitted after the application was filed and not included in this specification**

(73) Proprietor: **UNION CARBIDE CORPORATION**
**39 Old Ridgebury Road**
**Danbury Connecticut 06817 (US)**

(72) Inventor: **Ashworth, Robert William**
**106 Country Side Lane**
**Hackettstown New Jersey 07840 (US)**
Inventor: **Fu, Wallace Yam-Tak**
**804 Montrose Drive**
**South Charleston West Virginia 25303 (US)**

(74) Representative: **Baverstock, Michael George Douglas et al**
**BOULT, WADE & TENNANT 27 Furnival Street**
**London, EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the manufacture of thiobiscarbamates having the general formula:

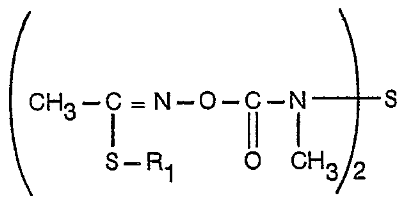

wherein $R_1$ is methyl, ethyl, n-propyl, isopropyl, n-butyl or isobutyl. These biscarbamate compounds are known to have pesticidal activity.

U.S. Patent No. 4004031 discloses a method of making these compounds involving simultaneous reaction of carbamate, base, and sulfur chloride ($SCl_2$ or $S_2Cl_2$). A disadvantage of this prior art method is its relatively low yield of products and high production of undesirable by-products.

EP—A—25354 describes and claims a method for making a compound of the formula

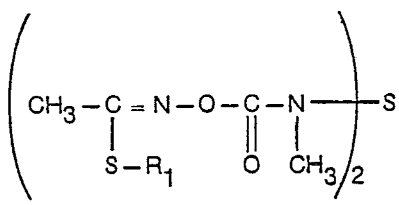

wherein $R_1$ is methyl, ethyl, n-propyl, isopropyl, n-butyl or isobutyl comprising the steps of:

(a) In presence of solvent, reacting nitrogen-containing heterocyclic base with a sulfur chloride selected from $SCl_2$, $S_2Cl_2$, and mixtures thereof to form a base-sulfur chloride adduct, and

(b) In presence of solvent, reacting the adduct with a carbamate of the formula

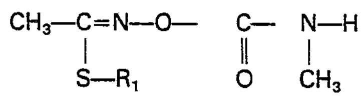

wherein $R_1$ is as previously defined. Said method produces thiobiscarbamates in higher yields and with less undesirable by-products than the method described in U.S. Patent No. 4004031. However, the stability of the products is not as high as might be wished.

It is an object of this invention to provide a method for making thiobiscarbamate compounds that produces higher yields.

It is another object of this invention to provide a method for making thiobiscarbamate compounds that produces less by-product.

These and other objects are attained by the presence invention, one aspect of which comprises a method for making a compound of the general formula:

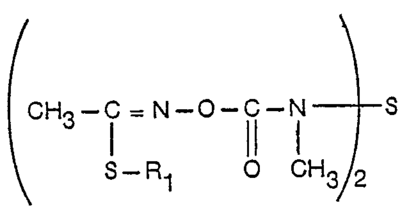

wherein $R_1$ is methyl, ethyl, n-propyl, isopropyl, n-butyl or isobutyl comprising the steps of:

(a) reacting pyridine with a sulfur chloride selected from $SCl_2$ or $S_2Cl_2$, or a mixture thereof to form a pyridine-sulfur chloride adduct, in the presence of excess pyridine,

(b) reacting the adduct with a carbamate of the general formula:

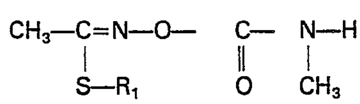

wherein $R_1$ is as previously defined, in the presence of pyridine, and

(c) purifying the product of step (b) by washing or slurrying the product at least once with water or a C—1 to C—5 alcohol.

The first step in making thiobiscarbamate compounds in accordance with the present invention is to react a nitrogen-containing heterocyclic base, specifically pyridine, and a sulfur chloride in the presence of excess pyridine to form a base-sulfur chloride adduct. This is believed to occur in accordance with the following equation:

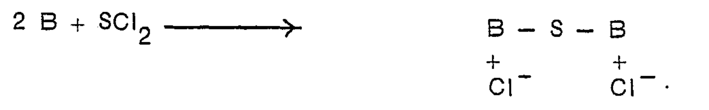

base sulfur dichloride adduct

or

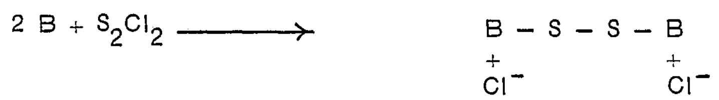

base sulfur mono chloride adduct

Preferably sufficient pyridine is used so that all of the sulfur chloride is reacted.

Sulfur dichloride is the preferred sulfur chloride. However, this compound decomposes slowly according to the equation

$$2SCl_2 \leftrightarrows S_2Cl_2 + Cl_2.$$

Hence commercial $SCl_2$, which currently is believed to contain roughly 70 to 85% $SCl_2$, is a practical and acceptable sulfur chloride. Sulfur monochloride, $S_2Cl_2$, is also acceptable, as are mixtures of $SCl_2$ and $S_2Cl_2$.

The reaction between the pyridine and the sulfur chloride is preferably carried out for about 0.25 to 1 hour at temperatures between about −10° and 50°C. More preferably the reaction temperature is about −10°C to 30°C and the reaction time about 20 min to 60 min.

The second step in the process is to react the adduct with a carbamate in the presence of pyridine as a solvent.

A preferred carbamate is methomyl. The reaction between methomyl and base-sulfur dichloride adduct is believed to proceed as follows:

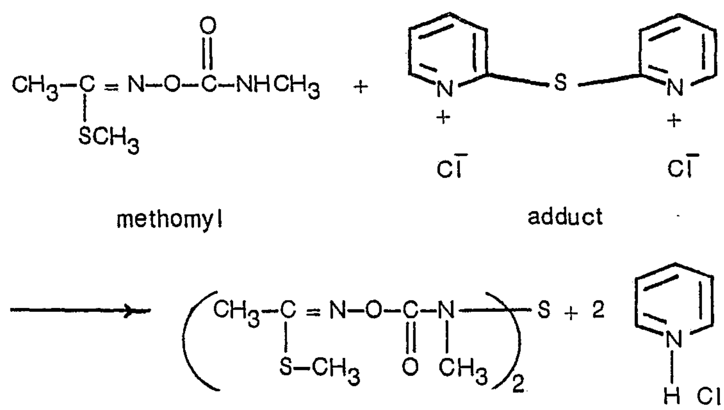

methomyl adduct

a preferred thiobiscarbamate

Since the first step yields an adduct-solvent mixture, preferably the second step is performed by adding carbamate to the adduct-solvent mixture resulting from the first step.

The second step is preferably carried out at a temperature of from about 0°C to 45°C, more preferably about 15°C to 32°C, and for a reaction time of about 1 hour to 96 hours, more preferably about 1 hour to 20 hours.

Purification of the product mixture from the second step is effected by washing and/or slurrying with water or a C—1 to C—5 alcohol.

## Example

The first step was performed as follows:

The adduct was formed by adding 23 grams of sulfur dichloride to 90 grams of pyridine dropwise over a period of about 15 minutes at 5°C. The adduct precipitated to form an adduct slurry.

The second step was performed as follows:

Into a separate reactor was placed 53.5 grams of methomyl and 60 grams of pyridine. The resulting mixture was stirred until the methomyl dissolved. The solution was maintained at a temperature of 20°C to 25°C.

The adduct slurry was pumped into the methomyl (pyridine solution with stirring while maintaining a reaction temperature of 20°C to 25°C. Adduct addition was completed after 1 hour. A reaction temperature of 20°C to 25°C was maintained for an additional four hours for the resulting mixture.

Purification of product was effected as follows: 300 grams of methanol was added to the above mixture to form a slurry. The slurry was then cooled to −5°C with stirring. The slurry was filtered to form a filter cake. The filter cake was washed with 150 grams of methanol at −5°C. The filter cake was then re-slurried in 150 grams of methanol at −5°C. The re-slurry was filtered and then washed twice with 150 grams of methanol at −5°C. The cake was dried in a vacuum oven at 40°C and 40 mm Hg. pressure. The cake consisted of 50.0 grams of product having a melting point of 169°C to 170°C.

**Claim**

A method for making a compound of the general formula:

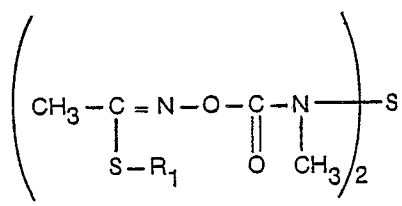

wherein $R_1$ is methyl, ethyl, n-propyl, isopropyl, n-butyl or isobutyl comprising the steps of:

(a) reacting pyridine with a sulfur chloride selected from $SCl_2$ or $S_2Cl_2$, or a mixture thereof to form a pyridine-sulfur chloride adduct, in the presence of excess pyridine,

(b) reacting the adduct with a carbamate of the general formula:

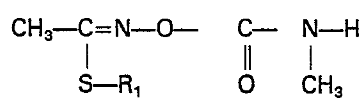

wherein $R_1$ is as previously defined, in the presence of pyridine, and

(c) purifying the product of step (b) by washing or slurrying the product at least once with water or a C—1 to C—5 alcohol.

**Patentanspruch**

Verfahren zur Herstellung einer Verbindung der allgemeinen Formel:

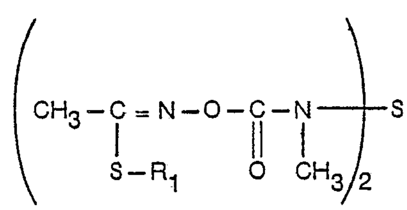

worin $R_1$ Methyl, Ethyl, n-Propyl, Isopropyl, n-Butyl oder Isobutyl ist, umfassend die Schritte:

(a) Umsetzen von Pyridin mit einem Schwefelchlorid, ausgewählt unter $SCl_2$ oder $S_2Cl_2$ oder einer Mischung davon, unter Bildung eines Pyridin-Schwefelchlorid-Addukts in Gegenwart von überschüssigem Pyridin,

(b) Umsetzen des Addukts mit einem Carbamat der allgemeinen Formel:

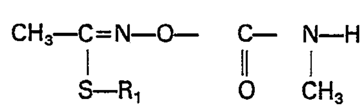

worin $R_1$ wie vorstehend definiert ist, in Gegenwart von Pyridin und

(c) Reinigen des Produkts von Stufe (b) durch mindestens einmaliges Waschen oder Aufschlämen des Produkts mit Wasser oder einem C—1 bis C—5-Alkohol.

**Revendication**

Procédé pour produire un composé de formule générale:

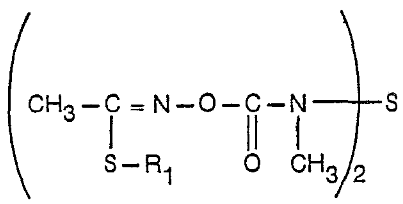

dans laquelle $R_1$ est un groupe méthyle, éthyle, n-propyle, isopropyle, n-butyle ou isobutyle, comprenant les étapes qui consistent:

(a) à faire réagir la pyridine avec un chlorure de soufre choisi entre $SCl_2$ et $S_2Cl_2$ ou leur mélange pour former un produit d'addition entre pyridine et chlorure de soufre, en présence d'une pyridine en excès,

(b) à faire réagir le produit d'addition avec un carbamate de formule générale:

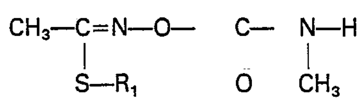

dans laquelle $R_2$ a la définition indiquée ci-dessus, en présence de pyridine, et

(c) à purifier le produit de l'étape (b) par son lavage ou sa mise en suspension au moins une fois avec de l'eau ou avec un alcool en $C_1$ à $C_5$.